Europäisches Patentamt

European Patent Office

Office européen des brevets

(11) Veröffentlichungsnummer: **0 401 525**
**A1**

(12) **EUROPÄISCHE PATENTANMELDUNG**

(21) Anmeldenummer: **90108615.7**

(51) Int. Cl.⁵· **A61M 19/00**

(22) Anmeldetag: **08.05.90**

(30) Priorität: **06.06.89 DE 3918431**

(43) Veröffentlichungstag der Anmeldung:
**12.12.90 Patentblatt 90/50**

(84) Benannte Vertragsstaaten:
**AT BE CH DE DK ES FR GB GR IT LI NL SE**

(71) Anmelder: **B. Braun Melsungen AG**
**Carl-Braun Strasse**
**D-3508 Melsungen(DE)**

(72) Erfinder: **Biscoping, Jürgen, Prof.Dr., Inst. für**
**Anästhesiol**

**Univ. Giessen, Klinikstrasse 10**
**D-6300 Giessen(DE)**
Erfinder: **Summerer, Marie-Louise**
**Buchenhain 15**
**D-3501 Körle(DE)**
Erfinder: **Witt, Hans-Hinrich, Dr.**
**Buchenhain 15**
**D-3501 Körle(DE)**

(74) Vertreter: **Selting, Günther, Dipl.-Ing. et al**
**Patentanwälte von Kreisler, Selting, Werner**
**Deichmannhaus am Hauptbahnhof**
**D-5000 Köln 1(DE)**

(54) **Katheterbesteck zur kontinuierlichen Spinalanästhesie.**

(57) Das Katheterbesteck weist eine Epiduralkanüle (10) auf, deren vorderes Ende (13) ohne schneidenden Schliff auf dem vorderen Ende stumpf ausgebildet ist. Zur Punktion ist ein Trokar (14) eingeschoben, der nur mit seiner Spitze (15) aus der Epiduralkanüle herausragt. Bei der Punktion wird das vordere Ende (13) gegen die Dura gelegt, ohne daß diese von der Spitze (15) durchstoßen würde. Danach kann ein zweiter Trokar eingeführt werden, der länger ist als der erste Trokar (14) und mit dem die Dura punktiert wird. Nach Entfernen des zweiten Trokars wird ein Katheter, die liegende Epiduralkanüle (10) als Führungkanüle nutzend, durch die punktierte Dura in den Spinalraum geschoben. Die durch den zweiten Trokar geschaffene durale Punktionsöffnung wird durch den Katheter vollkommen verschlossen. Diese Öffnung bleibt klein, da die Epiduralkanüle keinen die Dura schneidenden Schliff aufweist.

FIG.1

## Katheterbesteck zur kontinuierlichen Spinalanästhesie

Die Erfindung betrifft ein Katheterbesteck der im Oberbegriff des Patentanspruchs 1 angegebenen Art.

Bei der kontinuierlichen Spinalanästhesie wird ein Katheter von kleinem Durchmesser in den Spinalraum eingeführt. Probleme können durch die Punktion der Dura entstehen. Um Duraverletzungen und die daraus entstehenden postspinalen Kopfschmerzen so gering wie möglich zu machen, sollten Katheter mit möglichst kleinem Außendurchmesser verwendet werden. Andererseits erfordert bisher die Punktion der Dura die Verwendung einer Punktionskanüle, durch die anschließend der dünnere Katheter hindurchgeschoben und sodann die Punktionskanüle entfernt wird. Da das durch die Punktionskanüle verursachte Duraloch größer ist als der Katheterdurchmesser, fließt Liquor am Katheter vorbei durch das Duraloch. Die Folge ist ein hoher Liquorverlust mit der Möglichkeit postspinaler Kopfschmerzen.

DE 88 11 408 U1 beschreibt eine Kathetervorrichtung für die Spinalanästhesie, bei der eine Epiduralkanüle vorgesehen ist, welche bis zur Dura vorgeschoben wird. In die Epiduralkanüle kann eine Spinalkanüle eingeschoben werden, die länger ist als die Epiduralkanüle und die am vorderen Ende eine Spitze für die Durapunktion hat. In die Spinalkanüle wird zunächst ein Mandrin eingeführt, über dem, nach Entfernung der Spinalkanüle in Seldingertechnik anschließend der Katheter vorgeschoben wird. Der Katheter hat im wesentlichen denselben Durchmesser wie das erzeugte Duraloch, so daß die Größe des Duralochs auf das erforderliche Minimum beschränkt ist. Die oben beschriebene Kathetervorrichtung erfordert jedoch eine aufwendige Einführtechnik.

Der Erfindung liegt die Aufgabe zugrunde, ein Katheterbesteck der im Oberbegriff des Patentanspruchs 1 angegebenen Art zu schaffen, das in einfacher Weise gehandhabt werden kann und bei dem die Möglichkeit von Verletzungen der Dura auf das unbedingt erforderliche Maß beschränkt ist.

Die Lösung dieser Aufgabe erfolgt erfindungsgemäß mit den im kennzeichnenden Teil des Patentanspruchs 1 angegebenen Merkmalen.

Bei dem erfindungsgemäßen Katheterbesteck ist die Epiduralkanüle am vorderen Ende stumpf ausgebildet, d.h. sie weist keinen schneidenden Schliff auf, so daß sie stumpf gegen die Dura stößt, ohne diese zu beschädigen. Die Punktion erfolgt mittels eines Trokars, der in die Epiduralkanüle eingesteckt ist und nur mit seiner vorderen Spitze aus der Epiduralkanüle herausragt. Der Trokar weist eine kegelförmige Spitze und keine Schneide auf. Diese Spitze durchdringt das Fasergewebe von Ligamentum supra- und interspinale, ohne die Fasern zu zerschneiden. Ist die Epidurakanüle bis an das Ligamentum flavum gelangt, wird der Trokar herausgezogen und der Epiduralraum identifiziert, z.B. mittels "Loss of Resistance"-Methode. Nun wird die Epiduralkanüle um einige mm weiter vorgeschoben. Wenn sie an die Dura gelangt, durchdringt sie die Dura nicht, sondern sie legt sich an die Dura an und kann diese geringfügig vorschieben. Infolge des stumpfen vorderen Endes ist sie nicht imstande, in die Dura einzudringen, sondern sie bildet lediglich einen Führungskanal, der bis zur Dura hinführt. Das Punktieren der Dura erfolgt anschließend, wozu zweckmäßigerweise ein zweiter Trokar vorgesehen ist, der länger ist als der erste Trokar und im wesentlichen gleichen Durchmesser hat wie dieser. Dieser zweite Trokar, der ebenfalls eine nichtschneidende Spitze hat, wird durch die Dura nach intrathekal vorgeschoben und anschließend zurückgezogen, um danach den Katheter einführen zu können.

Eine zweite Möglichkeit besteht darin, in der bis an die Dura heranreichenden Epiduralkanüle einen Katheter vorzuschieben, in dem ein Mandrin sitzt, der mit seiner kegelförmigen Spitze um 1 mm bis 2 mm aus dem Katheter herausragt. Bei dem Vorschieben des Katheters stabilisiert der Drahtmandrin diesen und perforiert mit dem umschließenden Katheter die Dura.

In jedem Fall ist das Duraloch nicht größer als der Außendurchmesser des Katheters, so daß die Dura das den Katheter umgebende Duraloch eng umschließt und die aus tretende Liquormenge auf das unvermeidbare Maß reduziert wird.

Im folgenden wird unter Bezugnahme auf die Zeichnungen ein Ausführungsbeispiel der Erfindung näher erläutert.

Es zeigen:

Fig. 1 eine Seitenansicht der Epiduralkanüle, teilweise geschnitten, mit eingesetztem erstem Trokar,

Fig. 2 eine Seitenansicht der Epiduralkanüle, teilweise geschnitten, mit eingeführtem zweitem Trokar,

Fig. 3 einen Schnitt durch den zweiten Trokar,

Fig. 4 einen Längsschnitt durch die Epiduralkanüle mit eingeführtem Katheter,

Fign. 5 bis 8 die verschiedenen Stadien der Benutzung des Katheterbestecks, und

Fig. 9 eine andere Art der Benutzung des Katheterbestecks.

Das Katheterbesteck weist eine Epiduralkanüle 10 auf, die aus einem Kanülenrohr 11 und einem Griffstück 12 (oder Kanülenansatz) besteht. Das Kanülenrohr 11 ist an seinem vorderen Ende 13 stumpf abschließend ausgebildet, d.h. ohne einen schneidenden Schliff. Das vordere Ende 13 muß nicht unbedingt rechtwinklig zur Längsrich tung der Kanüle verlaufen, sondern es kann geringfügig schräggestellt sein, wenn die Kanüle dazu bestimmt ist, unter einem Winkel von weniger als 90° gegen die Dura eingeführt zu werden. Ferner kann das vordere Ende 13 abgerundet oder leicht konisch nach außen abgeschrägt sein. Das Kanülenrohr 11 hat z.B. Abmessungen von 0,7 x 0,9 x 80 mm, wobei die erste Zahl den Innendurchmesser, die zweite Zahl den Außendurchmesser und die dritte Zahl die freie Länge des Kanülenrohrs 11 angibt.

Der Kanal des Kanülenrohrs 11 erstreckt sich durch das Griffstück 12 hindurch. In die Epiduralkanüle 10 ist der erste Trokar 14 eingeführt. Dieser besteht aus einem Stab aus Vollmaterial, der an seinem vorderen Ende eine symmetrische Spitze 15 mit einer Länge von maximal etwa 2 mm aufweist. Bei vollständig eingeschobenem Trokar 14, also wenn das Griffstück 16 des Trokars gegen das Griffstück 12 der Epiduralkanüle stößt, überragt nur die Spitze 15 des Trokars das vordere Ende des Kanülenrohrs 11. Die Spitze 15 des Trokars kann auch beispielsweise pyramidenförmig ausgebildet sein oder einen asymmetrischen Schliff tragen.

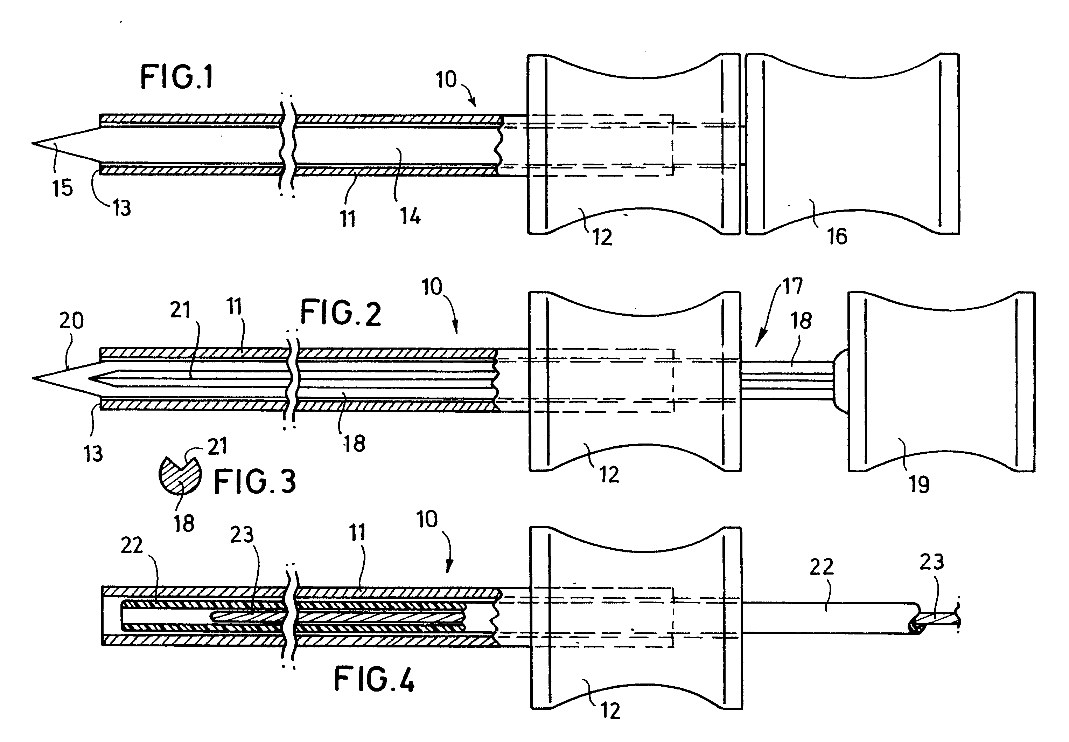

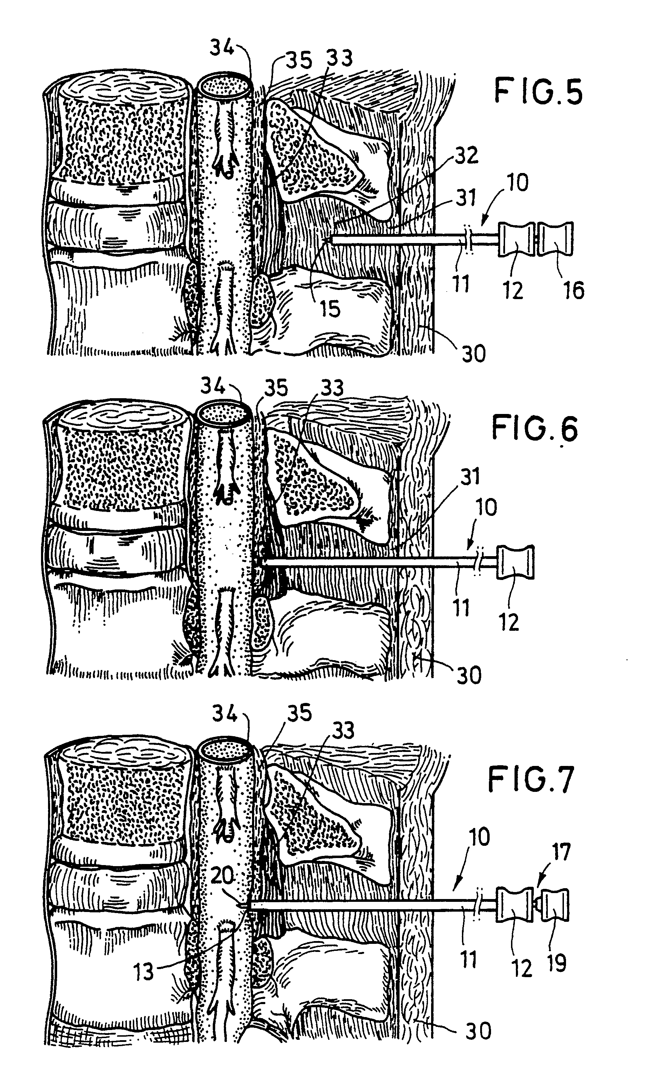

Die Vorrichtung wird in dem in Fig. 1 dargestellten Zustand gemäß Fig. 5 durch die Haut 30 hindurch in den Körper des Patienten eingeschoben, wobei die Spitze 15 der Punktion dient. Dabei wird zunächst das Ligamentum supraspinale 31 und dann das Ligamentum intraspinale 32 passiert, bis das Ligamentum flavum 33 erreicht wird. Der Trokar 14 wird entfernt und wenn der Anwender mit der Kanüle 10 den Periduralraum 35 identifiziert hat, wird die Vorrichtung bei einem Erwachsenen um weitere 5 mm bis 7 mm vorgeschoben (Fig. 6), um den Periduralraum 35 zu durchdringen. Dann liegt die stumpfe Spitze 13 mit großer Sicherheit an der Dura 34, die geringfügig vorgeschoben wird (Fig. 7).

Gemäß Fig. 2 ist ein zweiter Trokar 17 vorgesehen, der aus einem Vollstab 18 und einem Griffstück 19 besteht. Am vorderen Ende des Stabes 18 befindet sich eine symmetrische Spitze 20, die im Prinzip in gleicher Weise ausgebildet ist wie die Spitze 15 des ersten Trokars 14. Die Stichlänge des zweiten Trokars 17 ist um etwa 15 mm länger als die der Punktionskanüle 10 (und des ersten Trokars 14), so daß bei vollständig in die Epiduralkanüle eingestecktem zweitem Trokar 17 nicht nur die Spitze 20 aus dem Kanülenrohr 11 herausragt, sondern auch noch ein Abschnitt des Stabes 18.

Fig. 7 zeigt die Vorrichtung in dem in Fig. 2 dargestellten Zustand, nachdem der zweite Trokar 17 bis zum Endanschlag vorgeschoben worden ist, wobei die Spitze 20 die Dura durchstoßen hat.

Der zweite Trokar 17 weist einen durchgehenden längslaufenden Kanal 21 auf, der bei dem vorliegenden Fall als offene Rinne oder Kerbe mit einem Öffnungswinkel von 80° bis 90° ausgebildet ist. Durch diese Kerbe kann Liquor abfließen und somit den duralen Punktionserfolg anzeigen.

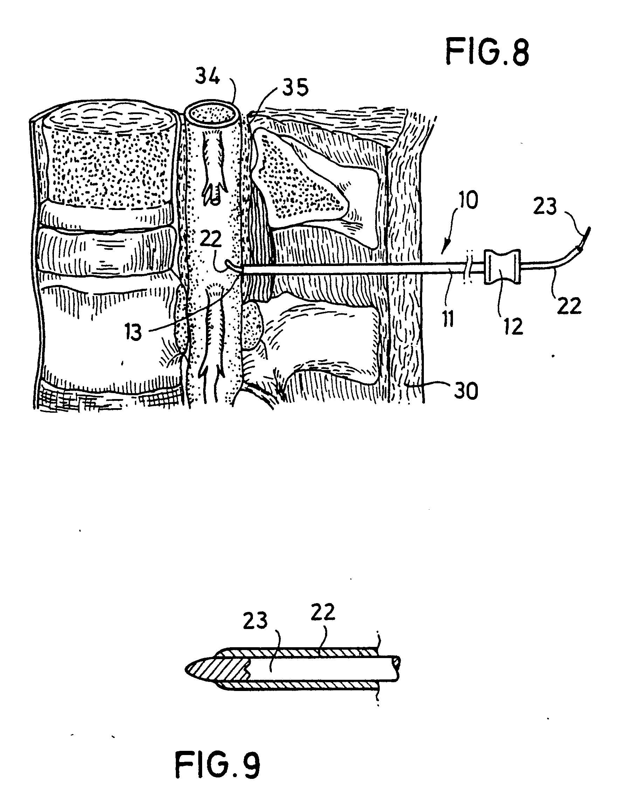

Nach erfolgter Punktion wird der zweite Trokar 17 aus der Epiduralkanüle 10 herausgezogen und der Katheter 22 mit dem darin befindlichen Mandrin 23 wird durch die Epiduralkanüle 10 hindurch in den Spinalraum hinein vorgeschoben (Fig. 8). Wenn die Steifigkeit des Katheters für das Vorschieben ausreicht, braucht ein Mandrin 23 nicht vorgesehen zu werden.

Die durch den zweiten Trokar 17 erzeugte durale Punktionsöffnung wird durch den Katheter 22 vollkommen ver schlossen. Diese Öffnung ist klein, da die Epiduralkanüle keinen die Dura schneidenden Schliff aufweist und diese nicht durchdringt.

Der Mandrin 23 des Spinalkatheters 22 beginnt 1 bis 2 mm hinter der vorderen Katheterspitze, so daß nur die relativ weiche Katheterspitze die Duraöffnung erreicht und durch diese hindurch in den Spinalraum gelangt. Der Mandrin 23 wird anschließend herausgezogen.

Fig. 8 zeigt das Einschieben des Katheters 22. Anschließend wird der Mandrin 23 herausgezogen und schließlich wird die Epiduralkanüle 10 über dem liegenden Katheter aus dem Körper des Patienten herausgezogen.

Für geübte Anwender ist die Benutzung des zweiten Trokars 17 nicht unbedingt erforderlich. Nach dem Auffinden des Periduralraumes 35 gemäß Fig. 6 wird gemäß Fig. 8 der Katheter 22 mit Mandrin 23 in die Kanüle eingeschoben, wobei die vordere Mandrinspitze gemäß Fig. 9 aus dem Katheter 22 heraus vorsteht. Die Durapunktion erfolgt in diesem Fall mit der vorstehenden Mandrinspitze. Nach erfolgter Durapunktion wird der Katheter 22 über dem Mandrin 23 vorgeschoben und anschließend werden Mandrin und Epiduralkanüle zurückgezogen.

## Ansprüche

1. Katheterbesteck zur kontinuierlichen Spinalanästhesie, mit einer Epiduralkanüle (10), einem in die Epiduralkanüle (10) einschiebbaren Punktionselement, dessen Spitze im eingeschobenen Zustand das vordere Ende (13) der Epiduralkanüle überragt, und einem durch die Epiduralkanüle (10) schiebbaren Katheter (22),

**dadurch gekennzeichnet,**

daß die Epiduralkanüle (10) am vorderen Ende (13) ohne schneidenden Schliff stumpf ausgebildet ist und daß das Punktionselement ein Trokar (14) ist, der im eingeschobenen Zustand das vordere Ende (13) der Epiduralkanüle (10) nur mit seiner vollen Spitze (15) überragt.

2. Katheterbesteck nach Anspruch 1, dadurch gekennzeichnet, daß ein zweiter Trokar (17) vorgesehen ist, der länger ist als der erste Trokar (14) und im wesentlichen gleichen Durchmesser hat wie dieser.

3. Katheterbesteck nach Anspruch 2, dadurch gekennzeichnet, daß der Katheter (22) einen Mandrin (23) enthält, der mit Abstand hinter dem vorderen Katheterende endet.

4. Katheterbesteck nach Anspruch 1, dadurch gekennzeichnet, daß der Katheter (22) einen Mandrin (23) enthält, der über das vordere Katheterende vorsteht und eine Spitze zur Punktion der Dura aufweist.

5. Katheterbesteck nach Anspruch 2, dadurch gekennzeichnet, daß der zweite Trokar (17) einen längslaufenden Liquorkanal (21) aufweist.

6. Katheterbesteck nach Anspruch 3, dadurch gekennzeichnet, daß der zweite Trokar (17) eine symmetrische, nichtschneidende Spitze (20) hat.

FIG.1

FIG.2

FIG.3

FIG.4

FIG.5

FIG.6

FIG.7

FIG.8

FIG.9

## EINSCHLÄGIGE DOKUMENTE

EP 90108615.7

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| A | DE - A1 - 2 405 918 (MARTINEZ-MANZOR) * Insgesamt, siehe besonders Seite 1, Absatz 1; Seite 2, letzer Absatz; Seite 3, letzter Absatz - Seite 4, Zeile 4; Seite 5, vorletzte Zeile - Seite 7, Absatz 1; Fig. 2,3 * -- | 1 | A 61 M 19/00 |
| A | DE - A1 - 3 508 013 (KREBS) * Seite 5, Zeilen 3-15; Seite 11, Zeilen 9-15; Seite 12, letzte Zeile - Seite 13, Zeile 5; Fig. 1 * -- | 1 | |
| A | DE - A1 - 3 248 067 (B. BRAUN MELSUNGEN AG) * Insgesamt, siehe besonders Seite 4, Zeilen 1-10; Seite 6, Zeile 1 - Seite 7, Zeile 7; Fig. 1 * ---- | | |

RECHERCHIERTE SACHGEBIETE (Int Cl⁵)

A 61 B 17/00
A 61 M  5/00
A 61 M 19/00

Der vorliegende Recherchenbericht wurde für alle Patentansprüche erstellt.

| Recherchenort WIEN | Abschlußdatum der Recherche 16-08-1990 | Prüfer VELINSKY-HUBER |
|---|---|---|